# EUROPEAN PATENT APPLICATION

(11) **EP 3 098 087 A1**
(43) Date of publication of application: **30.11.2016**
(21) Application number: 15468004.5
(22) Date of filing: 27.05.2015
(51) Int. Cl.: B43K 29/00, B43K 29/10, A61N 2/00, A61N 2/06, A61N 5/06

(54) **LIGHTING PEN**

(71) Applicant: Vivapen d.o.o., 3000 Celje (SI)
(72) Inventor: Melansek, Petra, 3000 Celje (SI); Laptev, Boris, 1000 Ljubljana (SI); Sidorenko, Galina, 1000 Ljubljana (SI)

(57) **Abstract**

The lighting pen consists of the body, light source with various colors of light, infra-red light sources and magnetic system. In the dark the user switches on the white light and infra-red light. In this case the white light goes outside through end face of the bottom part of the lighting pen.

The user may selectively switch on the sources of the red light arid infra-red light, or sources of blue light and infra-red light, or sources of green sources and infra-red light, or sources of white light and infra-red light.
In this case on a hand influences the visible light and infra-red light and a magnetic field with a small induction but with the significant gradients in a direction of line of magnetic field. The combination infra-red light, visible light and magnetic field has positive effect on physiological activity of the hand and reduces its weariness.

## Description

### FIELD OF THE INVENTION

The present invention relates to a writing instrument with an illumination, which provides the light for writing in the dark and at the same time has a positive influence on physiological activity of the hand and on the decreasing of its weariness.

### BACKGROUND OF THE INVENTION

Previous patents which describe self-illuminated pens with various colors of light are:
Patent US 5388038 A, Lighting pen, Shyue-Jong A. Yang,
Patent US 5845985 A, Night writer, Toungia Tei Xiong, Koua Xiong,
Patent US 3045111 A, Ball-point pen-light, Hoenig Sidney J.,
Patent US 6439734 B1, Pen light, Tien-Lin Lo.,
Patent US US6604880 B1, Motion lighting pen with light variably accompanying sound actuation, Fang-Yue Huang, Wen-Chen Chung,
Patent US D484535 S1, Lighting pen, Te Hsiang Fang,
Russian Patent No. 2017627, Lighting pen, Chepenko V.K. *http:*//*ru-patent. info*/*20*/*15*/*2017627. html*

In these patents the devices with illuminating are used only for writing in the dark. None of this patents has the information about a positive influence of these devices on physiological activity of the hand and on the decreasing of its weariness.

In US 8066397 B2, Light pen, Chih-Hsiung Lin the device with illuminating and 2 ring magnets are used. However magnets are located in parallel each other, on appreciable distance and are turned to each other with the same poles. Besides in the patent there is no information about positive influence of the device on physiological activity of the hand and on the decreasing of its weariness.

### SUMMARY OF THE INVENTION

The device with illuminating is not ideal in all respects. In particular, there is an ongoing need to increase the efficiency of working of the hand and fingers of the hand when a man writes a long time. About weariness of a hand when a man writes a long time, see

Russian Patent No. 2022799, Gorbunov A. E., Writing device of Gorbunov. *http:*//*ru-patent.info*/*20*/*20-2 4*/*2022799. html*

According to the invention, the device includes the illumination with four colors, infra-red light and magnetic field. When the device is in the hand, it influences on the hand simultaneously with the visible light, infra-red light and a magnetic field.

Magnetic system comprises at least two parallel magnets with small induction, located in plane and directed to each other and to a body with the opposite poles. At least two such magnets produce a significant gradients in a direction of line of magnetic field. It has been established in the literature that influence of magnetic field with significant gradients increases its positive effect and has a prophylactic effect, see
- Rubezhanskij K.A., Kolomiets A.A., Kataev G.A., etc. Using of magnetic apparatus for processing the water solutions (the manual for working professions).-M., 1980. - 76 P. http://www biblus.ru/Default.aspx?book=2e3g36ml
- Levitskij E.F., Laptev B.I.Sidorenko G.N. // Electromagnetic fields in balneology and physiotherapies. - Tomsk. - 2000. - 127 P. http://chamo.lib.tsu.ru/lib/item?id=chamo: 76516&theme =system#. VWHXu5qJg5s

Preferably, the distance between adjacent magnets of the magnet system is less than 1 mm, more preferably less than 0.05 mm.

In a preferred embodiment, the device comprises one or more light sources :
- one or more red light sources having a peak wavelength in the range of 600 nm to 790 nm,
- one or more green light sources having a peak wavelength in the range of 510 nm to 575 nm,
- one or more blue light sources having a peak wavelength in the range of 450 nm to 480 nm, and
- one or more infra-red light sources having a peak wavelength in the range of 790 nm to 1000 nm.
- one or more white light sources.

In a preferred embodiment, the device comprises a battery for powering the light sources.

In a preferred embodiment, the device further comprises a switching unit configured to selectively switch on a combination of the sources of the red light and infra-red light, or the sources of blue light and infra-red light, or the sources of green light and infra-red light, or the sources of white light and infra-red light.

It should be noted that the lighting pen with infra-red and visible light together with magnetic field is unknown.

It has been established that magnetic field improve microcirculation, favorably influence on physiological neogenesis, have antiinflammatory and antiedematous effect, see
- Illarionov V.E. Magnetotherapy . M.: The Book house «Librocom». 2009.-136 P. http://www.twirpx.com/file/1013474/

There is evidence in the literature that infra-red light and red light have good effects in prophylaxis and therapy and have a greater depth of penetration in living tissue. The red color restores a blood flow, metabolic processes, activates adrenals, improves immunity, etc. Infra-red light has antiinflammatory effects. Blue light has antibacterial and antiinflammatory effect, normalizes a condition of nervous system. Green light harmonizes processes in an organism and reduces a strain in nervous system, see
- Ulashchik V.S. Physiotherapy. Universal Medical Encyclopedia. - Mn.: Book House, 2008.-640 P. www.mrsu.ru/ru/getfile.php?ID=31729
- Kwon H.H., Lee J.B., Yoon J.Y. The clinical and histological effect of home-use, combination blue-red LED phototherapy for mild-to-moderate acne vulgaris in Korean patients: a double-blind, randomized controlled trial // Br J Dermatol. -2013.- V.168.-N5.- p.1088-1094.
- Papageorgiou P., Katsambas A., Chu A. Phototherapy with blue (415 nm) and red (660 nm) light in the treatment of acne vulgaris // Br J Dermatol. -2000.- V.142.,.N5.-p.973-978.
- Ishiguro M, Ikeda K, Tomita K. Effect of near-infrared light-emitting diodes on nerve regeneration //J Orthop Sci.- 2010.- V.15.- N2.- p.233-239.

The combination at this the invention the various colors of light, infra-red light and magnetic field with small induction but with a significant gradients in a direction of line of magnetic field has a positive influence on physiological activity of the hand and on the decreasing of its weariness.

### BRIEF DESCRIPTION OF THE DRAWINGS

The object and features of the present invention will become apparent from the following:

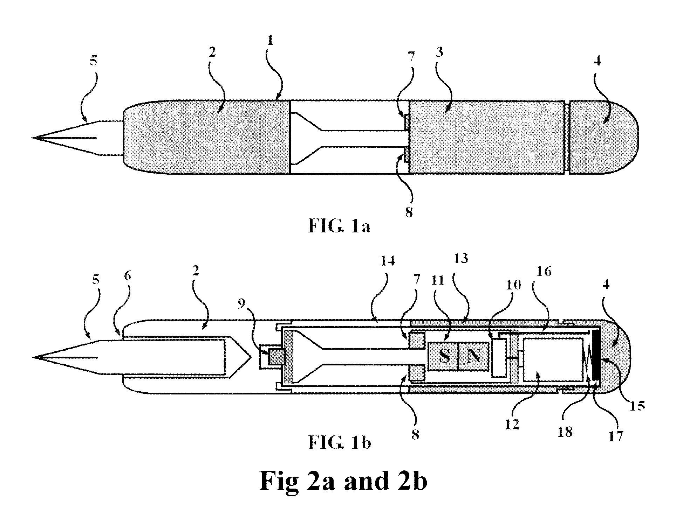

Fig. 1a is the whole view outside of a light pen 1. It has four compartments: a bottom compartment 2 which includes a writing element 5, a middle compartment of the body 3 which includes light sources, switching unit, magnetic system and upper compartment 4 which includes the switch.
Fig. 1b shows the detailed drawing of individual parts of a light pen.

### DESCRIPTION OF THE PREFERRED EMBODIMENT

Problem of the invention is to unite in the device an opportunity to write in darkness, in weak and good illumination and opportunity to make positive effect on physiological activity of the hand and to decrease its weariness.

Fig. 1a is the whole view of a light pen 1. It has four compartments. A bottom compartment 2 which includes a writing element 5, a middle compartment 3 which includes light sources, switching unit, magnetic system and upper compartment 4 which includes the switch.

Fig. 1b is the detailed drawing of individual parts. A bottom compartment 2 has an aperture 6 for an output the head of a writing element 5. The transparent bottom compartment 2 allows the light to pass through to a working zone, however bottom compartment 2 is covered outside with an opaque material for depression of illumination in darkness outside of a working zone.

A middle compartment 3 includes the light source with various colors of light 7, source of the infra-red light 8, source of white light 9, switching unit 10, magnetic system 11 and the battery 12. The upper part 13 of the middle compartment 3 is made of an opaque material, and the bottom part 14 of a middle compartment 3 is transparent.

The upper compartment 4 is made of an opaque material, includes the switch 15 for connection of the battery 12 to switching unit 10. The switch 15 consists of the first contact plate 16, which has contact with switching unit 10, and the second contact plate 17, which has contact with coiled spring 18 and battery 12.

When the upper compartment 4 comes near to the middle compartment 3 the first contact plate 16 and the second contact plate 17 are connected, and the current flows through the switching unit 10.

The switching unit 10 configured to selectively switch on a combination of the sources of the red light and infra-red light, or sources of blue light and infra-red light, or sources of green light and infra-red light, or sources of white light and infra-red light. In the dark the user switches the white light and infrared light. In this case, the light comes out through the end face of bottom compartment 2 of the lighting pen 1, but upon the hand of the user influence the infrared radiation and magnetic field.

### LIST OF REFERENCE SIGNS

- 1: light pen
- 2: bottom compartment
- 3: middle compartment
- 4: upper compartment
- 5: writing element
- 6: aperture
- 7: light source with different colors
- 8: source of the infra-red light
- 9: source of white light
- 10: switching unit
- 11: magnetic system
- 12: battery
- 13: upper part of the middle compartment
- 14: bottom part 14 of a middle compartment
- 15: switch
- 16: first contact plate
- 17: second contact plate
- 18: coiled spring

## Claims

1. A light pen device, comprising:
- hollow body and its compartments,
- writing element,
- the light source with various colors of light,
- switch.

2. The device of claim 1, wherein a bottom compartment of the hollow body is transparent, but is covered outside with an opaque material, and the bottom part of a middle compartment is transparent, but its upper part and the upper compartment are made of an opaque material.

3. The device of claim 2, wherein hollow body and its compartments are made of not magnetic material.

4. The device of claim 1, further comprises a switching unit, infra-red light sources and magnetic system.

5. The device of claim 4, a switching unit configured to selectively switch on a combination of the sources of the red light and infra-red light, or sources of blue light and infra-red light, or sources of green light and infra-red light, or sources of white light and infrared light.

6. The device of claim 5, wherein the sources of red, blue, green color and infra-red light are located at the upper part of a middle compartment, and the sources of white light are located at the bottom part of a middle compartment.

7. The device of claim 4, wherein the magnetic system consist of at least 2 magnets located in plane and directed to each other and to hand with the opposite poles.

8. The device of claim 4, wherein the distance between adjacent magnets is less than 1mm, preferably less than 0.05 mm.
